# EUROPEAN PATENT SPECIFICATION

(11) **EP 4 614 130 B1**
(45) Date of publication and mention of the grant of the patent: **25.11.2026**
(21) Application number: 25155257.6
(22) Date of filing: 31.01.2025
(51) Int. Cl.: G01M 3/32, H01M 10/42

(54) **SYSTEMS, APPARATUSES, AND METHODS FOR LEAK DETECTION USING A CAPILLARY TUBE**
SYSTEME, VORRICHTUNGEN UND VERFAHREN ZUR LECKDETEKTION UNTER VERWENDUNG EINES KAPILLARROHRS
SYSTÈMES, APPAREILS ET PROCÉDÉS DE DÉTECTION DE FUITE À L'AIDE D'UN TUBE CAPILLAIRE

(30) Priority: 06.03.2024 IN 202411015947
(43) Date of publication of application: 10.09.2025
(73) Proprietor: Honeywell International Inc., Charlotte, NC 28202 (US)
(72) Inventor: JAYAKUMAR, Prince Ashwin Kumar Anburaj, Charlotte, 28202 (US); KUMAR, Nirmal A., Charlotte, 28202 (US); MOHAPATRA, Bibhabasu, Charlotte, 28202 (US); RAO, Sharath N., Charlotte, 28202 (US)
(74) Representative: Haseltine Lake Kempner LLP

(56) References cited:
- CN-A- 111 337 201
- US-A- 5 212 979
- US-A1- 2022 034 746

## Description

### BACKGROUND

Liquid leak detection apparatuses may be used to detect various types of liquid leakages, for example leakage of a coolant of a battery pack. Some detection apparatuses use resistive sensors to detect a leakage, such as a coolant leakage. However, such detection apparatuses may suffer from false alarms. For example, they may detect water condensation as a coolant leakage. Through applied effort, ingenuity, and innovation, many of these identified problems have been solved by developing solutions that are included in embodiments of the present disclosure, examples of which are described in detail herein. Reference may be made to US 5 212 979 A wherein a testing device for measurement of a hydraulic leak rate of a spraying value with a closable outlet opening is discussed.
Reference may be made to CN 111 337 201 A wherein a leakage detection device is discussed.
Reference may be made to US 2022/034746 A1 wherein a method of detecting a leakage of a coolant in a battery cooling device for a vehicle is discussed.

### BRIEF SUMMARY

The present invention is defined by the appended independent claims, to which reference may now be made. Specific embodiments are defined in the dependent claims.

In one or more examples, the leak detection apparatus alternatively or additionally includes one or more computing devices, the one or more computing devices are further configured to determine a signature parameter for the liquid of interest, wherein the signature parameter comprises a viscosity difference between the liquid of interest and the other liquid and is determined using the measured air pressure in the capillary tube; and distinguish the liquid of interest from the other liquid using a capillary rise differentiation of the liquid of interest from the other liquid, wherein the capillary rise differentiation of the liquid of interest from the other liquid is based on the signature parameter.

In one or more examples, the leak detection apparatus alternatively or additionally includes one or more computing devices, the one or more computing devices are further configured to compare the measured air pressure in the capillary tube with an air pressure threshold; and determine a presence of the liquid of interest when the measured air pressure in the capillary tube is above the air pressure threshold.

In one or more examples, the leak detection apparatus alternatively or additionally includes a temperature sensor, wherein the temperature sensor comprises a temperature probe configured to engage with at least one of the liquid of interest or the other liquid in the spill collector, the temperature sensor configured to measure a temperature in the spill collector using the temperature probe.

In one or more examples, the leak detection apparatus alternatively or additionally includes one or more computing devices, the one or more computing devices are further configured to vary the air pressure threshold using the measured temperature.

In one or more examples, the leak detection apparatus alternatively or additionally includes one or more computing devices, the one or more computing devices are further configured to increase the air pressure threshold when the measured temperature is above a temperature threshold; and decrease the air pressure threshold when the measured temperature is below the temperature threshold.

In one or more examples, the leak detection apparatus alternatively or additionally includes one or more computing devices, the one or more computing devices are further configured to generate an alarm when the presence of the liquid of interest is determined.

In one or more examples, the liquid of interest is a coolant of a battery pack, the spill collector is a bottom cover of the battery pack, and wherein the other liquid is at least one of water generated by condensation, melted wax, or melted adhesive in the spill collector.

In some examples, a coolant leak detection system is described. In one or more example embodiments, the coolant leak detection system includes a spill collector configured to collect a coolant leaked from a battery pack. In one or more example embodiments, the coolant leak detection system alternatively or additionally includes a capillary tube including a first end configured to immerse in the coolant when leaked in the spill collector; and a second end configured to mechanically couple with a pressure sensor, the pressure sensor configured to measure an air pressure in the capillary tube above the coolant. In one or more example embodiments, the coolant leak detection system alternatively or additionally includes one or more computing devices electronically coupled with the pressure sensor, the one or more computing devices configured to distinguish the coolant from another liquid in the spill collector using the measured air pressure in the capillary tube.

In one or more example embodiments, the coolant leak detection system alternatively or additionally includes one or more computing devices, the one or more computing devices are further configured to determine a signature parameter for the coolant, wherein the signature parameter comprises a viscosity difference between the coolant and the other liquid and is determined using the measured air pressure in the capillary tube; and distinguish the coolant from the other liquid using a capillary rise differentiation of the coolant from the other liquid, wherein the capillary rise differentiation of the coolant from the other liquid is based on the signature parameter.

In one or more example embodiments, the coolant leak detection system alternatively or additionally includes one or more computing devices, the one or more computing devices are further configured to compare the measured air pressure in the capillary tube with an air pressure threshold; and determine a presence of the coolant when the measured air pressure in the capillary tube is above the air pressure threshold.

In one or more example embodiments, the coolant leak detection system alternatively or additionally includes a temperature sensor, wherein the temperature sensor comprises a temperature probe configured to engage with at least one of the coolant or the other liquid in the spill collector, the temperature sensor configured to measure a temperature in the spill collector using the temperature probe.

In one or more example embodiments, the coolant leak detection system alternatively or additionally includes one or more computing devices, the one or more computing devices are further configured to vary the air pressure threshold using the measured temperature.

In one or more example embodiments, the coolant leak detection system alternatively or additionally includes one or more computing devices, the one or more computing devices are further configured to increase the air pressure threshold when the measured temperature is above a temperature threshold; and decrease the air pressure threshold when the measured temperature is below the temperature threshold.

In one or more example embodiments, the coolant leak detection system alternatively or additionally includes one or more computing devices, the one or more computing devices are further configured to generate an alarm when the presence of the coolant is determined.

In some examples, the coolant is a coolant of a battery pack and the spill collector is a bottom cover of the battery pack, and wherein the other liquid is at least one of water generated by condensation, melted wax, or melted adhesive in the spill collector.

In one or more example embodiments, a leak detection method is described herein. In some example embodiments, the leak detection method includes measuring, by a pressure sensor, an air pressure in a capillary tube, wherein a first end of the capillary tube is configured to immerse in a liquid of interest in a spill collector configured to collect the liquid of interest from a leakage of the liquid of interest, and wherein the pressure sensor is mechanically coupled to a second end of the capillary tube. In some example embodiments, the leak detection method alternatively or additionally includes distinguishing, by one or more computing devices electronically coupled with the pressure sensor, the liquid of interest from another liquid using the measured air pressure in the capillary tube.

In some example embodiments, the leak detection method alternatively or additionally includes determining a signature parameter for the liquid of interest, wherein the signature parameter comprises a viscosity difference between the liquid of interest and the other liquid and is determined using the air pressure in the capillary tube; and distinguishing the liquid of interest from the other liquid using a capillary rise differentiation of the liquid of interest from the other liquid, wherein the capillary rise differentiation of the liquid of interest from the other liquid is based on the signature parameter.

In some example embodiments, the leak detection method alternatively or additionally includes comparing the measured air pressure in the capillary tube with an air pressure threshold; and determining a presence of the liquid of interest when the measured air pressure in the capillary tube is above the air pressure threshold.

In some example embodiments, the leak detection method alternatively or additionally includes measuring, using a temperature probe of a temperature sensor, a temperature in the spill collector; and varying the air pressure threshold using the measured temperature.

### BRIEF DESCRIPTION OF THE SEVERAL VIEWS OF THE DRAWINGS

FIG. 1 is a schematic diagram illustrating a liquid cooling system.
FIG. 2 is a schematic diagram illustrating a leak detection apparatus, in accordance with various embodiments.
FIG. 3 is a schematic diagram illustrating a leak detection apparatus, in accordance with various embodiments.
FIG. 4 is a schematic diagram illustrating one or more computing devices, in accordance with various embodiments.
FIG. 5 illustrates a method in accordance with various embodiments.
FIG. 6 illustrates a method in accordance with various embodiments.

### DETAILED DESCRIPTION

The phrases "in one embodiment," "according to one embodiment," "in some embodiments," "in various embodiments" and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

The word "example" or "exemplary" is used herein to mean "serving as an example, instance, or illustration" without a limitation. Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

Use of broader terms such as "comprises," "includes," and "having" should be understood to provide support for narrower terms such as "consisting of," "consisting essentially of," and "comprised substantially of". Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

The term "computing device" refers to any computer, controller (such as a microcontroller), processor, circuitry, and/or other executor of computer instructions that is embodied in hardware, software, firmware, and/or any combination thereof, that enables access to myriad functionalities associated with one or more mobile device(s), system(s), and/or one or more communications networks. Non-limiting examples of a computing device include a computer, a controller (such as a microcontroller), an application-specific integrated circuit, a field-programmable gate array, a personal computer, a smart phone, a laptop, a fixed terminal, a server, a networking device, a virtual machine, a processor, a plurality of processors electronically coupled to each other and placed in proximity of each other, remote from each other, in a various groups or bundles of one or more processors, and/or forming cloud computing or processing, etc. Other examples of computing devices are provided herein.

The term "electronically coupled," "electronically coupling," "electronically couple," "in electronic communication with," or "electronically connected" in the present disclosure refers to two or more elements or components being electronically connected, directly or indirectly. For example, two or more elements or components may be connected through wired means and/or wireless means, such that signals, voltage/current, data, information, or any other electronic signals may be transmitted to and/or received from these elements or components. Electronic connections established via an electrical connector and/or port may refer to wired connections.

The term "mechanically coupled" in the present disclosure refers to two or more mechanical elements (for example, but not limited to, a frame, a surface, a support unit, a joint, etc.) being physically connected in various ways such as directly, through intermediary elements, and/or using fastener(s), clasps, clamps, joints, pin joint, axle, hinge, adhesive, etc. The term "mechanically coupled" may refer to any of movable, turntable, swiveling, pivoting, fixed, and/or stationary mechanical coupling and/or any other similar type of mechanical coupling. In a non-limiting example, two components are mechanically coupled using a force, such as but not limited to magnetic force, force caused by air pressure, adhesive force, mechanical force, and/or other similar or related forces.

Battery packs have many technical applications such as in Electric Vehicles (EVs), robotics, household and/or industrial devices, energy distribution units, energy storage or emergency backup units for residential, healthcare, or commercial buildings, data servers, cellular base stations, etc. It is desirable to improve safety and/or reliability of battery packs.

In some examples, high temperatures may cause safety concerns for battery packs. In some examples, the temperature of a battery pack may increase due to the operation of the battery pack, for example due to current passing through internal conductive paths having electrical resistance in the battery cell, or due to low charging or discharging efficiency of the battery cells in the battery pack. If the increase in the temperature is not regulated, it may lead to a dangerous condition in the battery pack such as thermal runaway.

To regulate and keep the temperature within safe operating limits of a battery pack, various types of temperature regulating systems, apparatuses, and methods may be used. For example, a liquid cooling system may be used to regulate the temperature of a battery pack.

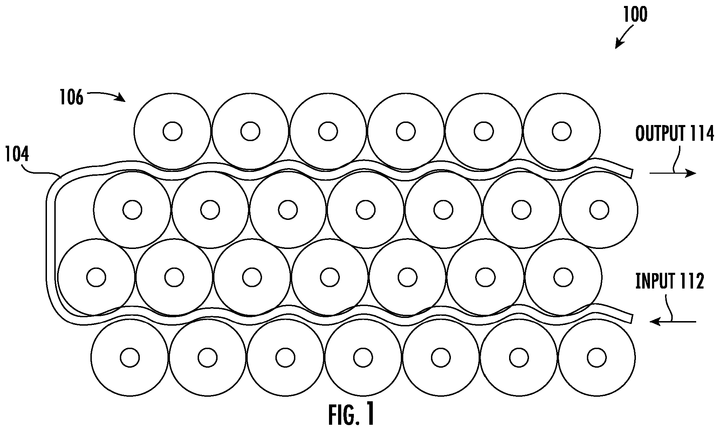

Referring now to FIG. 1 a schematic diagram illustrating a liquid cooling system 100 is provided in accordance with various embodiments of the present disclosure. The liquid cooling system 100 may circulate a coolant, e.g., a liquid coolant, through a tube 104 around the battery cells 106 of a battery pack to regulate the temperature. For example, coolant having a lower temperature is entered in the input 112 of the tube. The coolant may absorb heat from the battery cells 106 and the coolant with a higher temperature may exit from the output 114 of the tube.

However, there may be a leakage of the coolant from the tube due to a puncture, hole, cut, etc., in the tube. Leakage of the coolant may cause the temperature to increase in the battery pack which may have safety consequences as previously described. In some examples, increased temperatures may reduce efficiency of the battery pack. In some examples, coolant may cause a short circuit between the battery cells, causing increase in the idle load of the battery, which may reduce the efficiency of the battery pack and/or cause fire.

Various approaches have been used to detect leakage of a coolant. For example, a resistor sensor may be placed in the battery pack where the coolant may be spilled and/or accumulated due to a coolant leakage, for example in a spill collector (e.g., the bottom cover of the battery pack). When the resistor sensor detects a low resistance, it may be an indication of a leakage. However, this approach does not differentiate between a leakage caused by a coolant and condensation in the same location where the resistance sensor is place.

Various embodiments of the present disclosure provide methods, systems, and apparatuses to determine a leakage of the coolant while reducing false alarms caused by condensation.

The coolant may be spilled in the battery pack, for example in a spill collector of the battery pack. In various embodiments, a bottom cover of a battery pack may be the spill collector.

In various embodiments, a capillary tube is inserted in the battery pack. A capillary tube may be a tube that causes the coolant to rise in the tube due a capillary effect on the coolant in the spill collector. In various embodiments, the capillary effect refers to the rise of a liquid in the capillary tube due to the viscosity of the liquid. In various embodiments, one end of the capillary tube may be placed in the spill collector such that it is immersed in the spilled coolant once a leakage occurs. When a coolant leakage occurs, the coolant may rise in the capillary tube, for example due to the capillary effect.

In various embodiments, a diameter of the capillary tube is determined such that a capillary effect of interest is seen by liquids having a viscosity similar to the viscosity of the coolant. In example embodiments, because the viscosity of water is different from the viscosity of the coolant, the capillary tube will not have the same capillary effect on water as on the coolant. Hence, in example embodiments, water (for example water caused by condensation in the spill collector) will not have the same rise in the capillary tube as the coolant.

In various embodiments, due the capillary effect on the coolant, a leakage of coolant is determined and distinguished with a accumulated water in the spill collector. In various embodiments, the capillary effect on the coolant, due to the viscosity of the coolant, is determined to be a signature capillary effect of the coolant. Various embodiments determine a leakage of the coolant using the signature capillary effect of the coolant. For example, when there is water accumulation in the spill collector, there may be a low rise of water, or a negligible rise of water compared to a rise of coolant in the capillary tube.

In various embodiments, a pressure sensor determines the air pressure created in the capillary tube in case of a rise of a liquid in the capillary tube. In various embodiments, the air pressure is measured and used to determine whether a liquid risen in the capillary tube is a coolant, or another liquid with a different viscosity, such as water. In various embodiments, the pressure sensor is electronically coupled to one or more computing devices configured to determine a coolant leakage, generate an alarm and/or a coolant leakage detection signal when a rise of a coolant is detected in the capillary tube. By doing so, example embodiments distinguish between a leakage of coolant and an accumulation of other liquids, such as water, in the spill collector.

In various embodiments, a leakage of the coolant may be detected and/or differentiated from an accumulation of other liquids in the spill collectors, such as wax, melted wax, adhesives, etc., that may be used in the manufacturing of the battery packs.

Various embodiments of the present disclosure may be used to detect a liquid of interest. In various embodiments, a radius of the capillary tube is determined for the liquid of interest and using the viscosity of the liquid of interest, such that an amount of the rise by the liquid of interest in the capillary tube is unique for the liquid of interest. Consequently, in example embodiments, an air pressure created in the capillary tube as a result of the rise of the liquid in the capillary tube is unique for the liquid of interest. In example embodiments, by using the unique air pressure created in the capillary tube consequent to a rise of the liquid of interest in the capillary tube, a presence of the liquid of interest in the spill collector is determined.

In various embodiments, a radius for the capillary tube is selected such that a capillary rise (h) for the liquid of interest is above a height threshold. The capillary rise (h) for a liquid is determined according to the following equations: $h = \left(2∗T∗cos\left(θ\right)\right) / \left(ρ∗g∗r\right)$ wherein h is the capillary rise, T is a surface tension of the liquid, r is radius of the capillary tube, ρ is a density of the liquid, g is the acceleration due to gravity, and θ is an angle of contact.

In example embodiments, each liquid may have a unique capillary rise for a given temperature. For example, the viscosity of a liquid may change as the temperature of the liquid varies. In various embodiments, the change of the viscosity due to the variation in temperature is taken into account in detection of the liquid of interest.

In various embodiments, the detection of the liquid of interest includes thermal compensation. For example, the determination of the liquid of interest is compensated based on the temperature. In various embodiments, when the measured air pressure in the capillary sensor is above a threshold, a presence of the liquid of interest is determined. In various embodiments, the pressure threshold for determining the presence of the liquid of interest is adjusted based on the temperature. For example, the pressure threshold is increased when the temperature of the liquid in the spill collector increases, and the pressure threshold is decreased when the temperature of the liquid in the spill collector decreases.

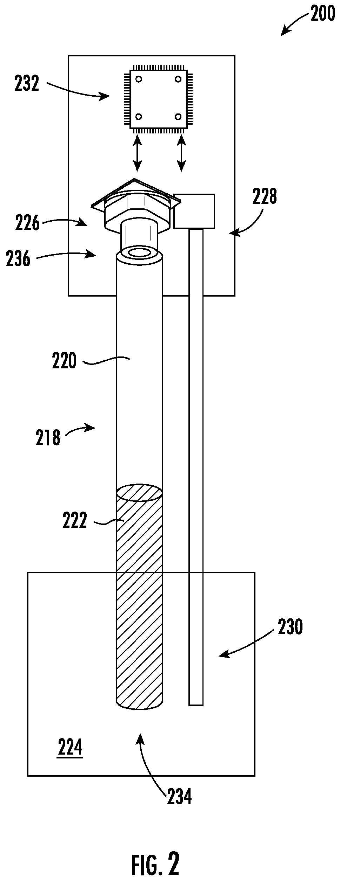

Referring now to FIG. 2 a schematic diagram illustrating a leak detection apparatus 200 is provided in accordance with various embodiments of the present disclosure.

In various embodiments, the leak detection apparatus 200 includes a spill collector 224. The spill collector 224 may be configured to collect a liquid of interest from a leakage of the liquid of interest. For example, the liquid of interest may be a coolant of a battery pack as previously described, and the spill collector 224 may be the bottom cover of the battery pack.

In various embodiments, the leak detection apparatus 200 includes a capillary tube 218. The capillary tube 218 includes a first end 234 configured to immerse in the liquid of interest in the spill collector 224. For example, when coolant has leaked and accumulated in the spill collector 224, the first end 234 will be immersed in the coolant.

In various embodiments, the capillary tube 218 includes a second end 236. The second end 236 may be configured to mechanically couple with a pressure sensor 226. In various embodiments, the pressure sensor 226 is configured to measure an air pressure of air 220 in the capillary tube above liquid 222.

In various embodiments, the capillary tube 218 is placed such that the first end 234 is immersed in a liquid gathered in spill collector 224. When a liquid gathers in the spill collector 224, due to viscosity of the liquid, it rises in the capillary tube 218. This rise of the liquid increases the air pressure of air 220 above the liquid 222. In various embodiments, the pressure sensor 226 is mechanically coupled in an airtight manner at the second end 236 of capillary tube 218.

In various embodiments, the leak detection apparatus 200 includes one or more computing devices 232. The one or more computing devices 232 may be electronically coupled with the pressure sensor 226. In various embodiments, the one or more computing devices 232 are configured to distinguish the liquid of interest from another liquid using the measured air pressure in the capillary tube. For example, due to condensation, water may be generated and accumulated in the spill collector 224. However, due to different viscosity of water and coolant, they rise to different levels in the capillary tube 218 causing different air pressures in air 220. For example, coolant may rise higher than water in capillary tube 218 causing a higher air pressure. In various embodiments, one or more computing devices 232 are configured to determine a presence of coolant in the first end 234 of the capillary tube. Hence a coolant leak may be detected when measured air pressure is above an air pressure threshold.

In various embodiments, the one or more computing devices 232 are configured to determine a signature parameter for the liquid of interest. In various embodiments, the signature parameter includes and/or is based on a viscosity of the liquid of interest. In various embodiments, the signature parameter includes and/or is based on a difference between the liquid of interest (e.g., coolant) and the other liquid (e.g., water) and is determined using the air pressure in the capillary tube.

In various embodiments, the one or more computing devices 232 are configured to distinguish the liquid of interest from the other liquid using a capillary rise differentiation of the liquid of interest from the other liquid. In various embodiments, the capillary rise differentiation of the liquid of interest from the other liquid is determined using the measured air pressure and/or a variation in the measure air pressure. In various embodiments, the capillary rise differentiation of the liquid of interest from the other liquid is based on the signature parameter. In various embodiments, the signature parameter of the liquid of interest is determined using the viscosity of the liquid of interest.

In various embodiments, the one or more computing devices 232 are configured to compare the measured air pressure in the capillary tube with an air pressure threshold. The one or more computing devices 232 may be configured to determine a presence of the liquid of interest when the measured air pressure in the capillary tube 218 is above the air pressure threshold.

In various embodiments, the leak detection apparatus 200 includes a temperature sensor 228. The temperature sensor 228 may include a temperature probe 230. The temperature probe 230 may be configured to engage with, or in contact with, or be inserted in at least one of the liquid of interest or the other liquid in the spill collector 224. In various embodiments, the temperature sensor 228 is configured to measure a temperature in the spill collector 224 using the temperature probe 230. For example, the temperature sensor 228 measures the temperature of the coolant and/or water accumulated in the spill collector 224.

The viscosity of a liquid may vary based on the temperature of the liquid. For example, the viscosity of a liquid may decrease when the temperature of the liquid increases and vie versa. Variation in viscosity, may change the capillary effect on the liquid. For example, a liquid with lower viscosity may rise higher in the capillary tube than when the temperature is lower.

To account for the variation of viscosity and capillary effect based on temperature, in various embodiments, the one or more computing devices 232 are further configured to vary the air pressure threshold using the measured temperature. For example, the one or more computing devices 232 are configured to increase the air pressure threshold when the measured temperature is above a temperature threshold. In various embodiments, the one or more computing devices 232 are configured to decrease the air pressure threshold when the measured temperature is below the temperature threshold.

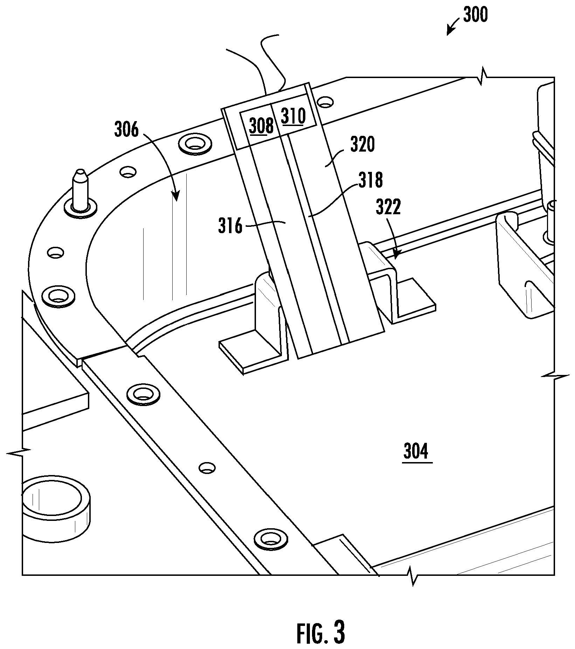

Referring now to FIG. 3 a schematic diagram illustrating leak detection system 300 is provided in accordance with various embodiments of the present disclosure.

In various embodiments, the leak detection system 300 determines a leak in a battery pack, for example a battery pack having liquid cooling system 100 with reference to FIG. 1. In various embodiments, the leak detection system 300 includes a spill collector. In various embodiments, the spill collector is a bottom cover 304 of the battery pack. For example, the battery pack may be used in an electric vehicle and the bottom cover 304 is a bottom cover of the battery pack of the electric vehicle.

In various embodiments, the spill collector is configured to collect a liquid of interest from a leakage of the liquid of interest. For example, the liquid of interest is the coolant used to reduce and/or regulate the temperature of a battery pack as previously described. In case of a leakage of the coolant, it is accumulated in the spill collector 306.

In various embodiments, the leak detection system 300 includes a capillary tube 218. The capillary tube may include a first end configured to immerse in the liquid of interest in the spill collector and a second end configured to mechanically couple with a pressure sensor 308. In various embodiments, the pressure sensor is configured to measure an air pressure in the capillary tube.

In various embodiments, one or more computing devices are electronically coupled with the pressure sensor. The one or more computing devices may be configured to distinguish the liquid of interest from another liquid using the measured air pressure in the capillary tube. For example, the one or more computing devices may distinguish coolant from water (e.g., generated due to condensation in the spill collector 306), melted wax and/or melted adhesive, or any other liquid that may be generated in the spill collector 306. In various embodiments, the one or more computing devices may use any of the techniques or methods described above to determine a presence of the liquid of interest and/or distinguish the liquid of interest from another liquid in the spill collector 306.

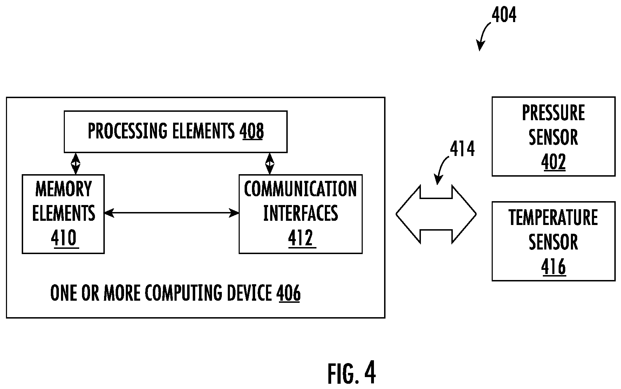

Referring now to FIG. 4 a schematic diagram illustrating one or more computing device 406 in communication with pressure sensor 402 and/or temperature sensor 416 is provided in accordance with various embodiments of the present disclosure.

In various embodiments, some or all of the one or more computing device 406 may be placed locally or in proximity with pressure sensor 402 and/or temperature sensor 416 (for example withing a same housing) or remotely from pressure sensor 402 and/or temperature sensor 416 (for example outside the housing). In various embodiments, the one or more computing device 406 may be placed inside another housing.

In various embodiments, the one or more computing device 406 is in communication with pressure sensor 402 and/or temperature sensor 416 or various other components of the leak detection apparatus or system such as a display, a vehicle control system, an alert system, etc., using communication interfaces 412 and over a wired and/or wireless medium 414. A wired medium 414 may be via any physical coupling of conductive materials, such as using wires, slip rings, linear sliding electrical connections, etc. A wireless medium 414 may include at least one of general packet radio service (GPRS), Universal Mobile Telecommunications System (UMTS), Code Division Multiple Access 2000 (CDMA2000), CDMA2000 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), IEEE 802.11 (Wi-Fi), Wi-Fi Direct, 802.16 (WiMAX), ultra-wideband (UWB), infrared (IR) protocols, near field communication (NFC) protocols, Wibree, Bluetooth protocols, Zigbee, wireless universal serial bus (USB) protocols, and/or any other wireless

In general, the terms computing apparatus, computer, system, device, entity, and/or similar words used herein interchangeably can refer to, for example, one or more computers, computing entities, desktops, mobile phones, tablets, notebooks, laptops, distributed systems, kiosks, input terminals, servers or server networks, blades, gateways, switches, processing devices, processing entities, controllers, control systems, set-top boxes, relays, routers, network access points, base stations, the like, and/or any combination of devices or entities adapted to perform the functions, operations, and/or processes described herein. Such functions, operations, and/or processes can include, for example, transmitting, receiving, operating on, processing, displaying, storing, determining, creating/generating, monitoring, evaluating, comparing, and/or similar terms used herein interchangeably. In one embodiment, these functions, operations, and/or processes can be performed on data, content, information, and/or similar terms used herein interchangeably. The one or more computing device 406 can include any computing device including, for example, a mobile device handling and/or processing apparatus configured to perform one or more steps/operations of one or more method or techniques described herein. In some embodiments, the one or more computing device 406 can include and/or be in association with one or more programmable logic controller (PLC), desktop computer(s), laptop(s), server(s), cloud computing platform(s), controller systems, and/or the like. In some example embodiments, the one or more computing device 406 can be configured to receive and/or transmit image processing instructions, data, and/or the like between the one or more leak detection apparatus or systems and/or their components to perform one or more steps/operations of one or more leak detection handling and/or processing techniques or methods described herein.

The one or more computing device 406 can include, or be in communications with, one or more processing elements 408 (also referred to as processors, processing circuitry, digital circuitry, and/or similar terms used herein interchangeably) that communicate with other elements within the one or more computing device 406 via a bus, for example. As will be understood, the processing elements 408 can be embodied in a number of different ways.

For example, the processing elements 408 can be embodied as one or more complex programmable logic devices (CPLDs), microprocessors, multi-core processors, coprocessing entities, application-specific instruction-set processors (ASIPs), microcontrollers, and/or controllers. Further, the processing elements 408 can be embodied as one or more other processing devices or circuitry. The term circuitry can refer to an entirely hardware embodiment or a combination of hardware and computer program products. Thus, the processing elements 408 can be embodied as integrated circuits, application specific integrated circuits (ASICs), field programmable gate arrays (FPGAs), programmable logic arrays (PLAs), hardware accelerators, digital circuitry, and/or the like.

As will therefore be understood, the processing elements 408 can be configured for a particular use or configured to execute instructions stored in volatile or non-volatile media or otherwise accessible to the processing elements 408. As such, whether configured by hardware or computer program products, or by a combination thereof, the processing elements 408 can be capable of performing steps or operations according to embodiments of the present disclosure when configured accordingly.

In one embodiment, the one or more computing device 406 can further include, or be in communication with, one or more memory elements 410. The one or more memory elements 410 can include non-volatile and/or volatile media. The memory elements 410, for example, can include non-volatile media (also referred to as non-volatile storage, memory, memory storage, memory circuitry and/or similar terms used herein interchangeably). In one embodiment, the non-volatile storage or memory can include one or more non-volatile storage or memory media, including, but not limited to, hard disks, ROM, PROM, EPROM, EEPROM, flash memory, MMCs, SD memory cards, Memory Sticks, CBRAM, PRAM, FeRAM, NVRAM, MRAM, RRAM, SONOS, FJG RAM, Millipede memory, racetrack memory, and/or the like.

As will be recognized, the non-volatile storage or memory media can store databases, database instances, database management systems, data, applications, programs, program modules, scripts, source code, object code, byte code, compiled code, interpreted code, machine code, executable instructions, and/or the like. The term database, database instance, database management system, and/or similar terms used herein interchangeably can refer to a collection of records or data that is stored in a computer-readable storage medium using one or more database models, such as a hierarchical database model, network model, relational model, entity- relationship model, object model, document model, semantic model, graph model, and/or the like.

In addition, or alternatively, the memory elements 410 can include volatile memory. For example, the one or more computing device 406 can further include, or be in communication with, volatile media (also referred to as volatile storage memory, memory storage, memory circuitry and/or similar terms used herein interchangeably). In one embodiment, the volatile storage or memory can also include one or more volatile storage or memory media, including, but not limited to, RAM, DRAM, SRAM, FPM DRAM, EDO DRAM, SDRAM, DDR SDRAM, DDR2 SDRAM, DDR3 SDRAM, RDRAM, TTRAM, TRAM, Z-RAM, RIMM, DIMM, SIMM, VRAM, cache memory, register memory, and/or the like.

As will be recognized, the volatile storage or memory media can be used to store at least portions of the databases, database instances, database management systems, data, applications, programs, program modules, scripts, source code, object code, byte code, compiled code, interpreted code, machine code, executable instructions, and/or the like being executed by, for example, the processing elements 408. Thus, the databases, database instances, database management systems, data, applications, programs, program modules, scripts, source code, object code, byte code, compiled code, interpreted code, machine code, executable instructions, and/or the like can be used to control certain aspects of the operation of the one or more computing device 406 with the assistance of the processing elements 408 and operating system.

As indicated, in one embodiment, the one or more computing device 406 can also include one or more communication interfaces 412 for communicating with various computing entities, such as by communicating data, content, information, and/or similar terms used herein interchangeably that can be transmitted, received, operated on, processed, displayed, stored, and/or the like. Such communication can be executed using a wired data transmission protocol, such as fiber distributed data interface (FDDI), digital subscriber line (DSL), Ethernet, asynchronous transfer mode (ATM), frame relay, data over cable service interface specification (DOCSIS), or any other wired transmission protocol. Similarly, the one or more computing device 406 can be configured to communicate via wireless external communication networks using any of a variety of protocols, such as general packet radio service (GPRS), Universal Mobile Telecommunications System (UMTS), Code Division Multiple Access 2000 (CDMA2000), CDMA2000 1X (1xRTT), Wideband Code Division Multiple Access (WCDMA), Global System for Mobile Communications (GSM), Enhanced Data rates for GSM Evolution (EDGE), Time Division-Synchronous Code Division Multiple Access (TD-SCDMA), Long Term Evolution (LTE), Evolved Universal Terrestrial Radio Access Network (E-UTRAN), Evolution-Data Optimized (EVDO), High Speed Packet Access (HSPA), High-Speed Downlink Packet Access (HSDPA), IEEE 802.11 (Wi-Fi), Wi-Fi Direct, 802.16 (WiMAX), ultra-wideband (UWB), infrared (IR) protocols, near field communication (NFC) protocols, Wibree, Bluetooth protocols, Zigbee, wireless universal serial bus (USB) protocols, and/or any other wireless protocol.

The one or more computing device 406 may perform various steps of various methods for detecting and/or distinguishing a leakage of the liquid of interest, in accordance with various embodiments of the present disclosure.

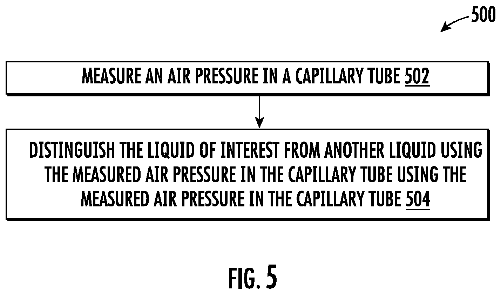

Referring now to FIG. 5 a schematic diagram illustrating a method 500 is provided in accordance with various embodiments of the present disclosure.

In various embodiments, in block 502, method 500 measures an air pressure in a capillary tube, for example capillary tube 218 or capillary tube 316. In various embodiments, method 500 measures the air pressure using a pressure sensor, for example pressure sensor 226 or pressure sensor 308 as previously described.

In various embodiments, a first end of the capillary tube is configured to immerse in a liquid of interest in a spill collector, for example spill collector 224 or spill collector 306. In various embodiments, the spill collector is configured to collect a liquid of interest from a leakage of the liquid of interest. For example, the spill collector is configured to collect coolant from a leakage of a coolant in a battery pack as for example shown in FIG. 1.

In various embodiments, the pressure sensor is mechanically coupled to a second end of the capillary tube. In various embodiments, the pressure sensor is mechanically coupled in an airtight way to the second end of the capillary tube.

In various embodiments, in block 504, method 500 distinguishes the liquid of interest from another liquid using the measured air pressure in the capillary tube using the measured air pressure in the capillary tube. In various embodiments, the method 500 uses the one or more computing devices electronically coupled with the pressure sensor to distinguish the liquid of interest from another liquid.

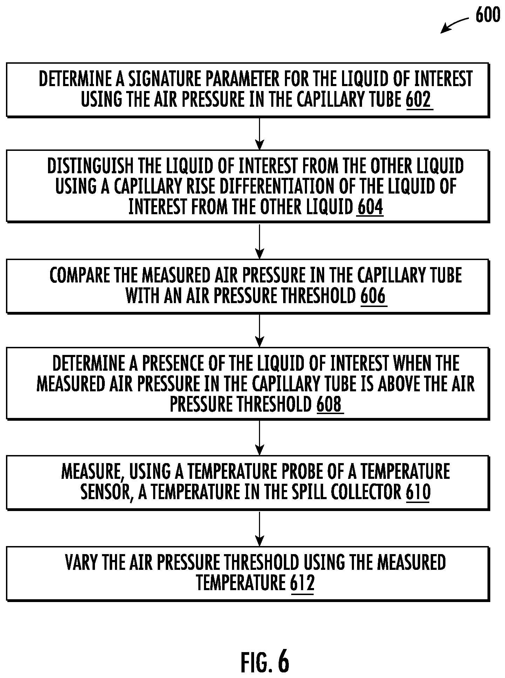

Referring now to FIG. 6 a schematic diagram illustrating a method 600 is provided in accordance with various embodiments of the present disclosure.

In various embodiments, in block 602, the method 600 determines a signature parameter for the liquid of interest. The signature parameter may include a viscosity difference between the liquid of interest and the other liquid and is determined using the air pressure in the capillary tube.

In various embodiments, in block 604, the method 600 distinguishes the liquid of interest from the other liquid using a capillary rise differentiation of the liquid of interest from the other liquid. The capillary rise differentiation of the liquid of interest from the other liquid may be based on the signature parameter.

In various embodiments, in block 606, the method 600 compares the measured air pressure in the capillary tube with an air pressure threshold. In various embodiments, at block 608, the method 600 determines a presence of the liquid of interest when the measured air pressure in the capillary tube is above the air pressure threshold.

In various embodiments, in block 610, the method 600 measures, using a temperature probe of a temperature sensor, a temperature in the spill collector at block 610. In various embodiments, in block 612 the method 600 varies the air pressure threshold using the measured temperature at block 612.

In various embodiments, method 600 increases the air pressure threshold when the measured temperature is above a temperature threshold. In various embodiments, method 600 decreases the air pressure threshold when the measured temperature is below the temperature threshold.

In various embodiments, method 600 uses one or more computing devices to perform various steps, such as determining a signature parameter, distinguishing the liquid of interest from another liquid, comparing the measure air pressure with the air pressure threshold, determining the presence of the liquid of interest, varying the air pressure threshold, generating an output and/or an alarm, etc.

In various embodiments, method 600 generates an alarm when the presence of the liquid of interest is determined. In various embodiments the liquid of interest is a coolant of a battery pack. In various embodiments, the spill collector is a bottom cover of the battery pack. In various embodiments, the other liquid is at least one of water generated by condensation, melted wax, or melted adhesive in the spill collector.

Although the example methods depict a particular sequence of operations, the sequence may be altered without departing from the scope of the present disclosure. For example, some of the operations depicted may be performed in parallel or in a different sequence that does not materially affect the function of the routine. In other examples, different components of an example device or system that implements the routine may perform functions at substantially the same time or in a specific sequence.

## Claims

1. A leak detection apparatus (200) comprising:
a spill collector (224) configured to collect a liquid of interest from a leakage of the liquid of interest;
a capillary tube (218) comprising:
a first end (234) configured to immerse in the liquid of interest in the spill collector (224); and
a second end (236) configured to mechanically couple with a pressure sensor (226), the pressure sensor (226) configured to measure an air (220) pressure in the capillary tube (218) based on a rise of the liquid of interest in the capillary tube (218), wherein the liquid of interest rises in the capillary tube (218) due to a viscosity of the liquid of interest; and
one or more computing devices (232) electronically coupled with the pressure sensor (226), the one or more computing devices (232) configured to distinguish the liquid of interest from another liquid using the measured air pressure in the capillary tube (218).

2. The leak detection apparatus (200) of claim 1, wherein the one or more computing devices (232) are further configured to:
determine a signature parameter for the liquid of interest, wherein the signature parameter comprises a viscosity difference between the liquid of interest and the other liquid and is determined using the measured air pressure in the capillary tube (218); and
distinguish the liquid of interest from the other liquid using a capillary rise differentiation of the liquid of interest from the other liquid, wherein the capillary rise differentiation of the liquid of interest from the other liquid is based on the signature parameter.

3. The leak detection apparatus (200) of claim 1, wherein the one or more computing devices (232) are further configured to:
compare the measured air pressure in the capillary tube (218) with an air pressure threshold; and
determine a presence of the liquid of interest when the measured air pressure in the capillary tube (218) is above the air pressure threshold.

4. The leak detection apparatus (200) of claim 3, further comprising a temperature sensor (228), wherein the temperature sensor (228) comprises a temperature probe (230) configured to engage with at least one of the liquid of interest or the other liquid in the spill collector (224), the temperature sensor (228) configured to measure a temperature in the spill collector (224) using the temperature probe (230).

5. The leak detection apparatus (200) of claim 4, wherein the one or more computing devices (232) are further configured to vary the air pressure threshold using the measured temperature.

6. The leak detection apparatus (200) of claim 4, wherein the one or more computing devices (232) are further configured to:
increase the air pressure threshold when the measured temperature is above a temperature threshold; and
decrease the air pressure threshold when the measured temperature is below the temperature threshold.

7. The leak detection apparatus (200) of claim 3, wherein the one or more computing devices (232) are further configured to generate an alarm when the presence of the liquid of interest is determined.

8. The leak detection apparatus (200) of claim 1, wherein the liquid of interest is a coolant of a battery pack, the spill collector (224) is a bottom cover (304) of the battery pack, and wherein the other liquid is at least one of water generated by condensation, melted wax, or melted adhesive in the spill collector (224).

9. A leak detection method comprising:
measuring, by a pressure sensor (226), an air pressure in a capillary tube (218), wherein a first end (234) of the capillary tube (218) is configured to immerse in a liquid of interest in a spill collector (224) configured to collect the liquid of interest from a leakage of the liquid of interest, and wherein the pressure sensor (226) is mechanically coupled to a second end (236) of the capillary tube (218), the pressure sensor (226) configured to measure the air (220) pressure in the capillary tube (218) based on a rise of the liquid of interest in the capillary tube (218), wherein the liquid of interest rises in the capillary tube (218) due to a viscosity of the liquid of interest; and
distinguishing, by one or more computing devices (232) electronically coupled with the pressure sensor (226), the liquid of interest from another liquid using the measured air pressure in the capillary tube (218).

10. The leak detection method of claim 9, further comprising:
determining a signature parameter for the liquid of interest, wherein the signature parameter comprises a viscosity difference between the liquid of interest and the other liquid and is determined using the air pressure in the capillary tube (218); and
distinguishing the liquid of interest from the other liquid using a capillary rise differentiation of the liquid of interest from the other liquid, wherein the capillary rise differentiation of the liquid of interest from the other liquid is based on the signature parameter.

11. The leak detection method of claim 10, further comprising:
comparing the measured air pressure in the capillary tube (218) with an air pressure threshold; and
determining a presence of the liquid of interest when the measured air pressure in the capillary tube (218) is above the air pressure threshold.

12. The leak detection method of claim 11, further comprising:
measuring, using a temperature probe (230) of a temperature sensor (228), a temperature in the spill collector (224); and
varying the air pressure threshold using the measured temperature.

## Patentansprüche

1. Leckdetektionseinrichtung (200), umfassend:
eine Austrittsauffangvorrichtung (224), die dazu konfiguriert ist, eine Flüssigkeit von Interesse aus einer Leckage der Flüssigkeit von Interesse aufzufangen;
ein Kapillarrohr (218), umfassend:
ein erstes Ende (234), das dazu konfiguriert ist, in die Flüssigkeit von Interesse in der Austrittsauffangvorrichtung (224) einzutauchen; und
ein zweites Ende (236), das dazu konfiguriert ist, sich mechanisch mit einem Drucksensor (226) zu koppeln, wobei der Drucksensor (226) dazu konfiguriert ist, einen Luft-(220) druck in dem Kapillarrohr (218) auf Basis eines Anstiegs der Flüssigkeit von Interesse in dem Kapillarrohr (218) zu messen, wobei die Flüssigkeit von Interesse aufgrund einer Viskosität der Flüssigkeit von Interesse in dem Kapillarrohr (218) ansteigt; und
eine oder mehrere Rechenvorrichtungen (232), die elektronisch mit dem Drucksensor (226) gekoppelt sind, wobei die eine oder die mehreren Rechenvorrichtungen (232) dazu konfiguriert sind, die Flüssigkeit von Interesse unter Verwendung des gemessenen Luftdrucks in dem Kapillarrohr (218) von einer anderen Flüssigkeit zu unterscheiden.

2. Leckdetektionseinrichtung (200) nach Anspruch 1, wobei die eine oder die mehreren Rechenvorrichtungen (232) ferner dazu konfiguriert sind:
einen Signaturparameter für die Flüssigkeit von Interesse zu bestimmen, wobei der Signaturparameter eine Viskositätsdifferenz zwischen der Flüssigkeit von Interesse und der anderen Flüssigkeit umfasst und unter Verwendung des gemessenen Luftdrucks in dem Kapillarrohr (218) bestimmt wird; und
die Flüssigkeit von Interesse von der anderen Flüssigkeit zu unterscheiden, unter Verwendung einer Kapillaranstiegsdifferenzierung der Flüssigkeit von Interesse gegenüber der anderen Flüssigkeit, wobei die Kapillaranstiegsdifferenzierung der Flüssigkeit von Interesse gegenüber der anderen Flüssigkeit auf dem Signaturparameter basiert.

3. Leckdetektionseinrichtung (200) nach Anspruch 1, wobei die eine oder die mehreren Rechenvorrichtungen (232) ferner dazu konfiguriert sind:
den gemessenen Luftdruck in dem Kapillarrohr (218) mit einem Luftdruckschwellenwert zu vergleichen; und
ein Vorhandensein der Flüssigkeit von Interesse zu bestimmen, wenn der gemessene Luftdruck in dem Kapillarrohr (218) über dem Luftdruckschwellenwert liegt.

4. Leckdetektionseinrichtung (200) nach Anspruch 3, ferner umfassend einen Temperatursensor (228), wobei der Temperatursensor (228) eine Temperatursonde (230) umfasst, die dazu konfiguriert ist, in mindestens eine von der Flüssigkeit von Interesse oder der anderen Flüssigkeit in der Austrittsauffangvorrichtung (224) einzugreifen, wobei der Temperatursensor (228) dazu konfiguriert ist, eine Temperatur in der Austrittsauffangvorrichtung (224) unter Verwendung der Temperatursonde (230) zu messen.

5. Leckdetektionseinrichtung (200) nach Anspruch 4, wobei die eine oder die mehreren Rechenvorrichtungen (232) ferner dazu konfiguriert ist, den Luftdruckschwellenwert unter Verwendung der gemessenen Temperatur zu variieren.

6. Leckdetektionseinrichtung (200) nach Anspruch 4, wobei die eine oder die mehreren Rechenvorrichtungen (232) ferner dazu konfiguriert sind:
den Luftdruckschwellenwert zu erhöhen, wenn die gemessene Temperatur über einem Temperaturschwellenwert liegt; und
den Luftdruckschwellenwert zu verringern, wenn die gemessene Temperatur unter dem Temperaturschwellenwert liegt.

7. Leckdetektionseinrichtung (200) nach Anspruch 3, wobei die eine oder die mehreren Rechenvorrichtungen (232) ferner dazu konfiguriert sind, einen Alarm zu erzeugen, wenn das Vorhandensein der Flüssigkeit von Interesse bestimmt wird.

8. Leckdetektionseinrichtung (200) nach Anspruch 1, wobei die Flüssigkeit von Interesse ein Kältemittel eines Akkus ist, die Austrittsauffangvorrichtung (224) eine Bodenabdeckung (304) des Akkus ist und wobei die andere Flüssigkeit mindestens eines von durch Kondensation erzeugtem Wasser, geschmolzenem Wachs oder geschmolzenem Kleber in der Austrittsauffangvorrichtung (224) ist.

9. Leckdetektionsverfahren, umfassend:
Messen, durch einen Drucksensor (226), eines Luftdrucks in einem Kapillarrohr (218), wobei ein erstes Ende (234) des Kapillarrohrs (218) dazu konfiguriert ist, in eine Flüssigkeit von Interesse in einer Austrittsauffangvorrichtung (224) einzutauchen, die dazu konfiguriert ist, die Flüssigkeit von Interesse aus einer Leckage der Flüssigkeit von Interesse aufzufangen, und wobei der Drucksensor (226) mechanisch mit einem zweiten Ende (236) des Kapillarrohrs (218) gekoppelt ist, wobei der Drucksensor (226) dazu konfiguriert ist, den Luft-(220) druck in dem Kapillarrohr (218) auf Basis eines Anstiegs der Flüssigkeit von Interesse in dem Kapillarrohr (218) zu messen, wobei die Flüssigkeit von Interesse aufgrund einer Viskosität der Flüssigkeit von Interesse in dem Kapillarrohr (218) ansteigt; und
Unterscheiden, durch eine oder mehrere Rechenvorrichtungen (232), die elektronisch mit dem Drucksensor (226) gekoppelt sind, der Flüssigkeit von Interesse von einer anderen Flüssigkeit unter Verwendung des gemessenen Luftdrucks in dem Kapillarrohr (218).

10. Leckdetektionsverfahren nach Anspruch 9, ferner umfassend:
Bestimmen eines Signaturparameters für die Flüssigkeit von Interesse, wobei der Signaturparameter eine Viskositätsdifferenz zwischen der Flüssigkeit von Interesse und der anderen Flüssigkeit umfasst und unter Verwendung des Luftdrucks in dem Kapillarrohr (218) bestimmt wird; und
Unterscheiden der Flüssigkeit von Interesse von der anderen Flüssigkeit, unter Verwendung einer Kapillaranstiegsdifferenzierung der Flüssigkeit von Interesse gegenüber der anderen Flüssigkeit, wobei die Kapillaranstiegsdifferenzierung der Flüssigkeit von Interesse gegenüber der anderen Flüssigkeit auf dem Signaturparameter basiert.

11. Leckdetektionsverfahren nach Anspruch 10, ferner umfassend:
Vergleichen des gemessenen Luftdrucks in dem Kapillarrohr (218) mit einem Luftdruckschwellenwert; und
Bestimmen eines Vorhandenseins der Flüssigkeit von Interesse, wenn der gemessene Luftdruck in dem Kapillarrohr (218) über dem Luftdruckschwellenwert liegt.

12. Leckdetektionsverfahren nach Anspruch 11, ferner umfassend:
Messen, unter Verwendung einer Temperatursonde (230) eines Temperatursensors (228), einer Temperatur in der Austrittsauffangvorrichtung (224); und
Variieren des Luftdruckschwellenwerts unter Verwendung der gemessenen Temperatur.

## Revendications

1. Appareil de détection de fuite (200) comprenant :
un collecteur de déversement (224) configuré pour collecter un liquide d'intérêt provenant d'une fuite du liquide d'intérêt ;
un tube capillaire (218) comprenant :
une première extrémité (234) configurée pour être immergée dans le liquide d'intérêt dans le collecteur de déversement (224) ; et
une deuxième extrémité (236) configurée pour se coupler mécaniquement à un capteur de pression (226), le capteur de pression (226) étant configuré pour mesurer une pression d'air (220) dans le tube capillaire (218) sur la base d'une montée du liquide d'intérêt dans le tube capillaire (218), dans lequel le liquide d'intérêt monte dans le tube capillaire (218) en raison d'une viscosité du liquide d'intérêt ; et
un ou plusieurs dispositifs informatiques (232) couplés électroniquement avec le capteur de pression (226), les un ou plusieurs dispositifs informatiques (232) étant configurés pour distinguer le liquide d'intérêt par rapport à un autre liquide en utilisant la pression d'air mesurée dans le tube capillaire (218).

2. Appareil de détection de fuite (200) selon la revendication 1, dans lequel les un ou plusieurs dispositifs informatiques (232) sont en outre configurés pour :
déterminer un paramètre signature pour le liquide d'intérêt, dans lequel le paramètre signature comprend une différence de viscosité entre le liquide d'intérêt et l'autre liquide et est déterminé en utilisant la pression d'air mesurée dans le tube capillaire (218) ; et
distinguer le liquide d'intérêt par rapport à l'autre liquide en utilisant une différenciation de montée capillaire du liquide d'intérêt de l'autre liquide, dans lequel la différenciation de montée capillaire du liquide d'intérêt de l'autre liquide est basée sur le paramètre signature.

3. Appareil de détection de fuite (200) selon la revendication 1, dans lequel les un ou plusieurs dispositifs informatiques (232) sont en outre configurés pour :
comparer la pression d'air mesurée dans le tube capillaire (218) à un seuil de pression d'air ; et
déterminer une présence du liquide d'intérêt lorsque la pression d'air mesurée dans le tube capillaire (218) est supérieure au seuil de pression d'air.

4. Appareil de détection de fuite (200) selon la revendication 3, comprenant en outre un capteur de température (228), dans lequel le capteur de température (228) comprend une sonde de température (230) configurée pour entrer en contact avec au moins un du liquide d'intérêt ou de l'autre liquide dans le collecteur de déversement (224), le capteur de température (228) étant configuré pour mesurer une température dans le collecteur de déversement (224) en utilisant la sonde de température (230).

5. Appareil de détection de fuite (200) selon la revendication 4, dans lequel les un ou plusieurs dispositifs informatiques (232) sont en outre configurés pour faire varier le seuil de pression d'air en utilisant la température mesurée.

6. Appareil de détection de fuite (200) selon la revendication 4, dans lequel les un ou plusieurs dispositifs informatiques (232) sont en outre configurés pour :
augmenter le seuil de pression d'air lorsque la température mesurée est supérieure à un seuil de température ; et
diminuer le seuil de pression d'air lorsque la température mesurée est inférieure au seuil de température.

7. Appareil de détection de fuite (200) selon la revendication 3, dans lequel les un ou plusieurs dispositifs informatiques (232) sont en outre configurés pour générer une alarme lorsque la présence du liquide d'intérêt est déterminée.

8. Appareil de détection de fuite (200) selon la revendication 1, dans lequel le liquide d'intérêt est un réfrigérant d'un bloc-batterie, le collecteur de déversement (224) est un capot inférieur (304) du bloc-batterie, et dans lequel l'autre liquide est au moins l'un parmi eau généré par condensation, cire fondue ou adhésif fondu dans le collecteur de déversement (224).

9. Procédé de détection de fuite comprenant :
la mesure, par un capteur de pression (226), d'une pression d'air dans un tube capillaire (218), dans lequel une première extrémité (234) du tube capillaire (218) est configurée pour être immergée dans un liquide d'intérêt dans un collecteur de déversement (224) configuré pour collecter le liquide d'intérêt provenant d'une fuite du liquide d'intérêt, et dans lequel le capteur de pression (226) est couplé mécaniquement à une deuxième extrémité (236) du tube capillaire (218), le capteur de pression (226) étant configuré pour mesurer la pression d'air (220) dans le tube capillaire (218) sur la base d'une montée du liquide d'intérêt dans le tube capillaire (218), dans lequel le liquide d'intérêt monte dans le tube capillaire (218) en raison d'une viscosité du liquide d'intérêt ; et
la distinction, par un ou plusieurs dispositifs informatiques (232) couplés électroniquement au capteur de pression (226), du liquide d'intérêt par rapport à un autre liquide en utilisant la pression d'air mesurée dans le tube capillaire (218).

10. Procédé de détection de fuite selon la revendication 9, comprenant en outre :
la détermination d'un paramètre signature pour le liquide d'intérêt, dans lequel le paramètre signature comprend une différence de viscosité entre le liquide d'intérêt et l'autre liquide et est déterminé en utilisant la pression d'air dans le tube capillaire (218) ; et
la distinction du liquide d'intérêt par rapport à l'autre liquide en utilisant une différenciation de montée capillaire du liquide d'intérêt de l'autre liquide, dans lequel la différenciation de montée capillaire du liquide d'intérêt de l'autre liquide est basée sur le paramètre signature.

11. Procédé de détection de fuite selon la revendication 10, comprenant en outre :
la comparaison de la pression d'air mesurée dans le tube capillaire (218) à un seuil de pression d'air ; et
la détermination d'une présence du liquide d'intérêt lorsque la pression d'air mesurée dans le tube capillaire (218) est supérieure au seuil de pression d'air.

12. Procédé de détection de fuite selon la revendication 11, comprenant en outre :
la mesure, en utilisant une sonde de température (230) d'un capteur de température (228), d'une température dans le collecteur de déversement (224) ; et
la variation du seuil de pression d'air en utilisant la température mesurée.
